# EUROPÄISCHE PATENTANMELDUNG

(11) **EP 0 876 812 A2**
(43) Veröffentlichungstag der Anmeldung: **11.11.1998**
(21) Anmeldenummer: 98107385.1
(22) Anmeldetag: 23.04.1998
(51) Int. Cl.: A61K 7/48

(54) **Zuckersäureureide enthaltende kosmetische Zubereitungen**

(30) Priorität: 06.05.1997 DE 19719006
(71) Anmelder: BASF AKTIENGESELLSCHAFT, 67056 Ludwigshafen (DE)
(72) Erfinder: Wekel, Hans-Ulrich, Dr., 67158 Ellerstadt (DE); von dem Bussche-Hünnefeld, Linda, Dr., 68623 Lampertheim (DE); Streicher, Harald, Dr., 67069 Ludwigshafen (DE); Westenfelder, Horst, 67435 Neustadt (DE)

(57) **Zusammenfassung**

Verwendung von Zuckersäureureiden der allgemeinen Formel I, 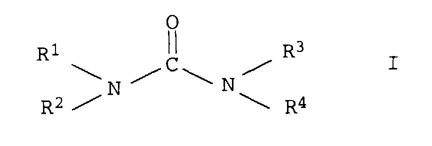 in der die Variablen folgende Bedeutung haben:
- R¹: ein Zuckersäurerest
- R²: Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können,
- R³, R⁴: unabhängig voneinander Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder einen Zuckersäurerest,
als Wirkstoff in der Kosmetik sowie Zuckersäureureide enthaltende kosmetische Zubereitungen und Verfahren zu deren Herstellung.

## Beschreibung

Die Erfindung betrifft Zuckersäureureide, deren Verwendung als Wirkstoffe in der Kosmetik sowie ein Verfahren zu deren Herstellung.

Zu den wichtigsten Aufgaben der Haut gehört es, den Körper vor dem Eindringen und vor dem Verlust von Stoffen zu schützen. Eine intakte Haut ist leicht feucht, glatt und geschmeidig. Die Vermeidung einer sofortigen Abdunstung des Schweißes sind die wichtigsten Faktoren des Schutzes der Haut vor Austrocknung. Wie in SÖFW-Journal, 122. Jahrgang, 14, S. 998-999 (1996) beschrieben, zählen zu den Schutzfaktoren vor allem hygroskopische Substanzen in der Hornzelle, wie Zucker, Harnstoff und das Oberflächenfett.

Aus "Kosmetik" [Georg Thieme Verlag Stuttgart, 2. Auflage (1995), Herausgeber: W. Umbach] ist zu entnehmen, daß kosmetische Hautpflegemittel häufig wasserbindende Substanzen als Feuchthaltemittel (Moisturizer) enthalten. Hierzu zählen Polyole wie Glycerin und Sorbit, ethoxylierte Polyole und hydrolysierte Proteine. Weitere Anwendung finden Komponenten des natürlichen Feuchthaltefaktors der Haut (Natural Moisturizing Factor), wie die oben genannte Substanzen beispielsweise Harnstoff oder bestimmte Aminosäuren.

Aufgabe der Erfindung war es, Harnstoffderivate für kosmetische Zwecke vorzuschlagen, mit denen der Feuchtigkeitshaushalt der Haut positiv beeinflußt werden kann.

Diese Aufgabe wurde erfindungsgemäß gelöst durch Verwendung von Zuckersäureureiden der allgemeinen Formel I, 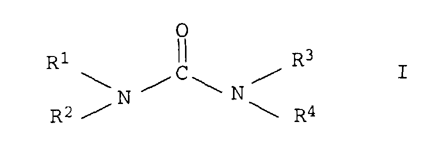 in der die Variablen folgende Bedeutung haben:
- R¹: Zuckersäurerest
- R²: Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können,
- R³, R⁴: unabhängig voneinander Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder einen Zuckersäurerest,
für kosmetische Zubereitungen.

Bei den als Kosmetikwirkstoff zu verwendenden, erfindungsgemäßen Zuckersäureureiden handelt es sich um Verbindungen von Harnstoff bzw. Harnstoffderivaten mit C₄- bis C₆-Zuckersäuren sowie deren Keto-Derivaten. Eine Zusammenfassung der zu verwendenden Zuckersäuren findet sich in "The Monosaccharides" (J. Stanek, Academic Press, 1963, S. 664 bis 666, S. 695 bis 727). Bevorzugt verwendete Zuckersäuren sind C₅- und C₆-Aldonsäuren sowie C₅- und C₆-Ketoaldonsäuren, insbesondere Zuckersäuren aus der Gruppe Ribonsäure, Arabonsäure, Gluconsäure, Galactonsäure, Glucuronsäure, Galacturonsäure, Gulonsäure und Keto-gulonsäure.

Die Hydroxylfunktionen der erfindungsgemäß verwendeten Zuckersäuren können in ungeschützter oder geschützter Form vorliegen, wobei im letzteren Fall prinzipiell alle aus der Zuckerchemie bekannten Schutzgruppen wie beispielsweise Acetyl, Benzoyl, Pivaloyl, Benzyl, 4-Methoxybenzoyl, tert.-Butyldimethylsilyl, Trimethylsilyl, Benzyliden, 4-Methoxybenzyliden, Isopropyliden geeignet sind. Weitere einsetzbare Schutzgruppen sind von T.W. Greene und P.G.M. Wuts in "Protective Groups in Organic Synthesis" (John Wiley & Sons, Inc., 1991), Seite 10ff beschrieben.

Die Erfindung betrifft auch die neuen Verbindungen der Formel Ia, 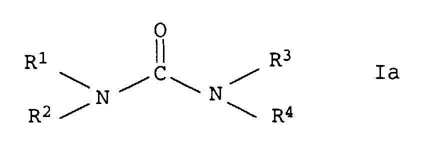 in der R¹ für einen Zuckersäurerest aus der Gruppe Ribonsäure, Arabonsäure, Gluconsäure, Galactonsäure, Glucuronsäure, Galacturonsäure, Gulonsäure und Keto-gulonsäure steht, R² Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl bedeutet, wobei die drei letztgenannten Reste substituiert sein können und R³ sowie R⁴ unabhängig voneinander für Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder für einen Zuckersäurerest aus der Gruppe Ribonsäure, Arabonsäure, Gluconsäure, Galactonsäure, Glucuronsäure, Galacturonsäure, Gulonsäure und Keto-gulonsäure stehen.

Bereits bekannte Zuckersäureureide, wie 2-Ureido-D-ribo-hexulofuranosonamid, beschrieben in Biosci. Biotech. Biochem., 60(7), 1198-1200, 1996 sowie 2-Keto-D-gluconsäureureid (Tetrahedron, 52(4), 1177-1194, 1996) und die in WO 94/13685 beschriebenen Ureide der Psicofuranose bzw. Psicopyranose dienen als Vorstufen für die Synthese von Hydantocidin, einem potentiell wirksamen Herbizid. Die Verwendung dieser Zuckersäureureide in der Kosmetik ist bis dato nicht bekannt.

Bei den erfindungsgemäßen Zuckersäureureiden der Formel I und Ia sind als Acylreste für R² bis R⁴ verzweigte oder unverzweigte, gesättigte oder ungesättigte, gegebenenfalls mehrfach ungesättigte C₁-C₂₀-Acylketten, insbesondere Formyl, Acetyl, Propionyl, n-Butyryl, iso-Butyryl, n-Valeryl, Palmitoyl sowie die entsprechenden Acylreste der Sorbin-, Öl-, Linol- oder Linolensäure zu verstehen.

Als Alkylreste R² bis R⁴ seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste R² bis R⁴ seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Cycloalkylreste seien für R² bis R⁴ bevorzugt verzweigte oder unverzweigte C₃-C₁₀-Cycloalkylketten wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt.

Als Cycloalkenylreste seien für R² bis R⁴ bevorzugt verzweigte oder unverzweigte, C₃-C₁₀-Cycloalkenylketten mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecyl genannt.

Die Cycloalkenyl- und Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Die Erfindung betrifft außerdem ein Verfahren zur Herstellung von Zuckersäureureiden der Formel I, dadurch gekennzeichnet, daß man eine aktivierte Zuckersäure mit Harnstoff oder einem Harnstoffderivat der Formel II, 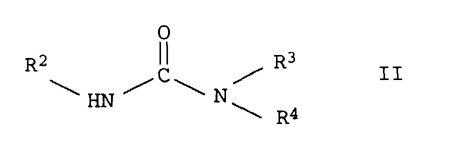 in der R² Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl bedeutet, wobei die drei letztgenannten Reste substituiert sein können, und R³ sowie R⁴ unabhängig voneinander für Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder für einen Zuckersäurerest aus der Gruppe Ribonsäure, Arabonsäure, Gluconsäure, Galactonsäure, Glucuronsäure, Galacturonsäure, Gulonsäure und Keto-gulonsäure stehen, umsetzt.

Die Herstellung der bereits bekannten Zuckersäureureide erfolgt durch aufwendige, mehrstufige Synthesen. So wird in Tetrahedron 52(4), 1177-1194, 1996 das Ureid der Psicofuranose, ausgehend von der korrespondierenden Säure, durch eine mehrstufige Synthese wie folgt hergestellt:
1. Umsetzung der Säure zum Amid,
2. in situ Synthese des N-Acylisocyanats durch Reaktion mit Oxalylchlorid,
3. Umsetzung mit Ammoniak zum Ureid.

Umso überraschender war es daher, daß sich die Zuckersäuren, beispielsweise 2,3:4,6-Di-O-isopropyliden-2-keto-L-gulonsäure nach Aktivierung z.B. zum Säurechlorid mit Harnstoff erfindungsgemäß zum entsprechenden Ureid umsetzten lassen.

Unter einer aktivierten Carbonsäure versteht man Derivate, bei denen die Reaktivität der Carboxylfunktion erhöht ist. Typische Vertreter aktivierter Carbonsäuren sind unter anderen Säurechloride, -anhydride, -imidazolide sowie Carbonsäureazide. Daneben lassen sich Carbonsäuren auch in Gegenwart von Carbodiimiden aktivieren. Beispiele aktivierter Carbonsäuren sowie deren Reaktionen sind in J. March "Advanced Organic Chemistry", J. Wiley & Sons, 4. Auflage, 1992, S. 392-396 und S. 417-421 zusammengefaßt.

Die Aktivierung der Zuckersäure verläuft bevorzugt über die Stufe des Zuckersäurechlorids. Dabei werden die Zuckersäure und das Chlorierungsmittel in Molverhältnissen von 1 : 0,4 bis 1 : 10, bevorzugt 1:1 bis 1:3 umgesetzt. Als Chlorierungsmittel können beispielsweise PCl₅, PCl₃, POCl₃, Oxalylchlorid oder insbesondere SOCl₂ verwendet werden.

Die Reaktion wird im allgemeinen in für Chlorierungsreaktionen geeigneten Lösungsmitteln, insbesondere in polaren aprotischen Lösungsmitteln wie z.B. Dimethylformamid, Dioxan, Pyridin [siehe dazu H. Bosshard, Helv. Chim. Acta 42 (1959) 1653-1658], aber auch in chlorierten Kohlenwasserstoffen wie z.B. Dichlormethan in einem weiten Temperaturintervall von -50°C bis +150°C, insbesondere von -30°C bis +40°C durchgeführt. Besonders bevorzugt ist der Temperaturbereich von -20°C bis +30°C. Es ist auch möglich, die Chlorierung mit Thionylchlorid ohne Zugabe eines weiteren Lösungsmittels durchzuführen. In diesem Fall erfolgt die Reaktion in einem Temperaturintervall von -20°C bis 100°C, bevorzugt bei der Siedetemperatur von SOCl₂.

Die Reaktionszeit hängt unmittelbar von der Reaktionstemperatur ab. Im allgemeinen ist die Reaktion nach 2 bis 48 Std., bevorzugt nach 12 bis 24 Std. beendet.

Das entstandene Zuckersäurechlorid kann entweder isoliert oder aber in einer bevorzugten Ausführungsvariante direkt mit Harnstoff oder Harnstoffderivaten der Formel II weiter umgesetzt werden.

Die Umsetzung des Zuckersäurechlorids mit Harnstoff oder Harnstoffderivaten der Formel II erfolgt ebenfalls in den oben erwähnten Lösungsmitteln in einem weiten Temperaturintervall von -50°C bis +100°C, insbesondere von -30°C bis +50°C. Besonders bevorzugt ist hier der Temperaturbereich von -20°C bis +30°C.

Es ist aber auch möglich, die Umsetzung von Harnstoff oder Harnstoffderivaten der Formel II mit Zuckersäurechloriden in Gegenwart von Pyridin oder anderen tertiären Basen, die den Halogenwasserstoff binden, wie beispielsweise Trimethylamin, Triäthylamin oder Dimethylaminopyridin durchzuführen

Nach Beendigung der Reaktion wird das Reaktionsgemisch gegebenenfalls durch Zugabe einer Base, beispielsweise NaHCO₃ neutralisiert, hydrolysiert und aufgearbeitet.

Die Abspaltung gegebenenfalls vorhandener Zuckerschutzgruppen erfolgt nach den üblichen, in der Literatur bekannten Verfahren. Eine Übersicht der gängigsten Abspaltungsreaktionen findet sich in T.W. Greene und P.G.M. Wuts in "Protective Groups in Organic Synthesis" (John Wiley & Sons, Inc., 1991).

Die erfindungsgemäßen Verbindungen I sind als Wirkstoffe, vor allem als Feuchthaltemittel, für kosmetische Anwendungen geeignet.

Kosmetische Präparate oder Zubereitungen enthalten die Verbindungen (I) im allgemeinen zu 0,1 bis 15 Gew.-%, vorzugsweise zu 0,3-5 Gew.%, bezogen auf die Gesamtmenge der kosmetischen Formulierung, neben den in der Kosmetik üblichen Trägerstoffen oder Verdünnungsmitteln und gegebenenfalls üblichen kosmetischen Hilfsstoffen.

Von der Art des Trägers, Hilfsstoffes oder Verdünnungsmittel hängt es ab, ob das fertige, Zuckersäureureid-haltige Präparat eine Lösung, ein Öl, eine Creme, eine Salbe, eine Lotion, ein Gel oder ein Pulver ist. Derartige Zubereitungen können beispielsweise der Zeitschrift "Seifen, Öle, Fette, Wachse", 1955, Seite 147 sowie Hagers Handbuch der pharmazeutischen Praxis, Springer Verlag, Band 2 (1991), Kapitel 4 entnommen werden.

Üblicherweise verwendete kosmetische Hilfsstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel und Parfüms.

Beispiele für kosmetische Öle sind pflanzliche Öle, wie Erdnußöl, Jojobaöl, Olivenöl, Sesamöl, Baumwollsamenöl, Kokosöl, Mandelöl, Traubenkernöl, Ricinusöl oder Mineralöle, synthetische Fettsäureester und Glyceride. Grundlage für Salben sind beispielsweise Vaseline, Lanolin, Eucerin oder Polyethylenglykole.

Grundlagen für Cremes können sowohl Emulgatoren vom Wasser- in-Öl-Typ, wie z.B. Wollfett, Sorbitanester und Monoglyceride als auch Öl-in-Wasser-Emulgatoren, wie Natriumseifen, Natriumsalze von Schwefelsäureestern einiger Fettalkohole oder Polysorbate sein.

In den nachfolgenden Beispielen wird die Herstellung der erfindungsgemäßen Zuckersäureureide sowie die Zuckersäureureide-enthaltenden kosmetischen Formulierungen näher erläutert.

### Beispiel 1

### N-Aminocarbonyl-2,3:4,6-di-O-isopropyl-L-ketogulonsäureamid

80 g (292 mmol) 2,3:4,6-Di-O-isopropyl-L-ketogulonsäure wurden in 500 ml absol. Dimethylformamid gelöst. Bei -10 °C wurden 42.6 ml (584 mmol) Thionylchlorid innerhalb von 10 min. zugetropft. Die Lösung wurde bei Raumtemperatur 20 std. gerührt. Anschließend wurde auf -10 °C gekühlt und 17.5 g (292 mmol) Harnstoff zugegeben. Die Lösung wurde bei Raumtemperatur 20 std. nachgerührt.

Danach wurde mit NaHCO₃ neutralisiert, zu 2 l H₂O gegossen und mit CH₂Cl₂ extrahiert. Die vereinigten organischen Extrakte wurden mit gesättigter NaCl-Lösung und H₂O gewaschen, über Na₂SO₄ getrocknet, filtriert und eingeengt.

Das erhaltene Rohprodukt wurde aus Essigester umkristallisiert.
Ausbeute: 37.9 g (41 %)
Schmelzpunkt: 190 - 191°C
¹H-NMR (CDCl₃) : 8.95 (1H, NH) ; 7.99, 5.87 (2H, NH₂) ; 4.58, 4.40, 4.23 (3H, H-3, H-4, H-5); 4.17 (2H, H-6); 1.58, 1.56, 1.42, 1.39 (12H, 4 x CH₃) ppm.

### Bespiel 2

### N-Aminocarbonyl-2,3-O-isopropyl-L-ketogulonsäureamid

18.8 g (59.3 mmol) N-Aminocarbonyl-2,3:4,6-di-O-isopropyl-L-ketogulonsäureamid wurden in 1 l H₂O mit 10 g DOWEX 50Wx8 (H⁺) 30 min. bei 70°C gerührt. Anschließend wurde der Ionentauscher abfiltriert und das Filtrat durch Gefriertrocknung eingeengt. Das erhaltene Rohprodukt wird an Kieselgel gereinigt.
Ausbeute: 14.9 g (91 %)
¹H-NMR (d₆-DMSO): 9.18 (1H, NH); 7.58, 7.47 (2H, NH₂); 4.85, 4.69 (2H, 2 x OH), 4.55, 4.20, 4.07, 4.12 (3H, H-3, H-4, H-5); 3.65 (2 x H-6); 1.47, 1.32 (6H, 2 x CH₃) ppm.

### Beispiel 3

### N-Aminocarbonyl-L-ketogulonsäureamid

2 g N-Aminocarbonyl-2,3-O-isopropyl-L-ketogulonsäureamid wurden in 16 ml Trifluoressigsäure und 2 ml H₂O 3 Std. bei Raumtemperatur gerührt. Anschließend wurde Toluol zugegeben und eingeengt. Das nach mehrfachem Codestillieren erhaltene Rohprodukt wurde chromatographisch aufgereinigt.
Ausbeute: 1.4 g (82%)

### Kosmetische Zubereitungen

### Beispiel 4

### Zusammensetzung für fettfreies Sonnenschutz-Gel

| Massengehalt (Gew.-%) | |
|---|---|
| 0,40 | Acrylat/C₁₀-C₃₀ Alkylacrylat Crosspolymer |
| 0,25 | Hydroxyethyl Cellulose |
| 8,00 | Methoxyzimtsäure-octylester |
| 1,00 | 4-Methylbenzyliden Campher |
| 0,50 | N-Aminocarbonyl-2,3-O-isopropyl-L-ketogulonsäureamid |
| 0,20 | Dinatrium EDTA |
| ad 100 | Wasser |
| 5,00 | Glycerin |
| 0,15 | Fragrance |
| 0,30 | Imidazolydinyl Harnstoff |
| 0,25 | Natrium Methylparaben |
| 0,15 | Natrium Propylparaben |
| 5,00 | PEG-25 PABA |
| 0,10 | Natriumhydroxyd |

### Beispiel 5

### Zusammensetzung für Feuchtigkeitscreme

| Massengehalt (Gew.-%) | |
|---|---|
| 6,00 | PEG-7-Hydrogenated Castor Oil |
| 5,00 | Isopropyl Palmitat |
| 6,00 | Mineralöl |
| 5,00 | Jojobaöl |
| 5,00 | Mandelöl |
| 0,50 | Tocopherolacetat |
| 2,00 | N-Aminocarbonyl-2,3-O-isopropyl-L-ketogulonsäureamid |
| 0,60 | Magnesiumstearat |
| 2,00 | PEG-45 / Dodecyl Glycol Copolymer |
| 5,00 | Glycerin |
| 0,25 | Methylparaben |
| 0,15 | Propylparaben |
| 5,00 | Imidazolydinyl Harnstoff |
| 0,15 | Fragrance |
| 0,20 | Dinatrium EDTA |
| ad 100 | Wasser |

### Beispiel 6

### Zusammensetzung für Nachtcreme ohne Konservierungsmittel

| Massengehalt (Gew.-%) | |
|---|---|
| 5,00 | PEG-7-Hydrogenated Castor Öl |
| 4,00 | Isopropylpalmitat |
| 4,00 | Caprylsäure/Caprinsäure Triglycerid |
| 3,00 | N-Aminocarbonyl-2,3-O-isopropyl-L-ketogulonsäureamid |
| 1,50 | PEG-45/Dodecyl Glycol Copolymer |
| 0,50 | Magnesiumstearat |
| 1,50 | Dimethicon |
| 4,00 | 1,2 Propylenglycol |
| 4,00 | Glycerin |
| 8,00 | 611 Alkohol |
| ad 100 | Wasser |
| 2,00 | Kollagen |
| 0,15 | Fragrance |

### Beispiel 7

### Zusammensetzung für Antifaltencreme

| Massengehalt (Gew.-%) | |
|---|---|
| 6,00 | PEG-7-Hydrogenated Castoröl |
| 5,00 | Isopropylpalmitat |
| 10,00 | Mineralöl |
| 3,00 | Caprylsäure/Caprinsäure Triglycerid |
| 0,60 | Magnesiumstearat |
| 1,00 | N-Aminocarbonyl-2,3-O-isopropyl-L-ketogulonsäureamid |
| 1,50 | Tocopherolacetat |
| 2,00 | PEG-45/Dodecyl Glycol Copolymer |
| 0,05 | Tocopherol |
| 0,20 | Retinol |
| ad 100 | Wasser |
| 0,30 | Glycerin |
| 0,70 | Magnesiumsulfat |
| 0,25 | Methylparaben |
| 0,15 | Propylparaben |
| 0,20 | Natriumascorbylmonophosphat |
| 0,10 | α-Tocopherol |
| 0,10 | Ascorbylpalmitat |
| 0,15 | Fragrance |

### Beispiel 8

### Zusammensetzung für Feuchtigkeits Tagescreme

| Massengehalt (Gew. -%) | |
|---|---|
| 2,00 | Cremophor A 6 |
| 2,00 | Cremophor A 25 |
| 10,00 | Mineralöl |
| 3,00 | Caprylsäure/Caprinsäure Triglycerid |
| 3,00 | Isostearinsäure |
| 3,00 | N-Aminocarbonyl-2,3-O-isopropyl-L-ketogulonsäureamid |
| 1,50 | Tocopherolacetat |
| 2,00 | D-Panthenol USP |
| 0,05 | Tocopherol |
| 0,20 | Retinol |
| ad 100 | Wasser |
| 0,30 | Glycerin |
| 0,15 | Dibromocyanobutan |
| 0,20 | Natriumascorbylmonophosphat |
| 0,10 | α-Tocopherol |
| 0,10 | Ascorbylpalmitat |
| 0,15 | Fragrance |

## Patentansprüche

1. Verwendung von Zuckersäureureiden der allgemeinen Formel I, 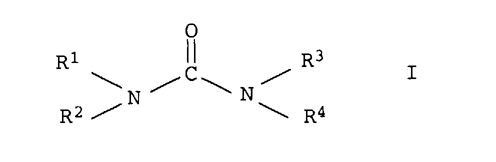 in der die Variablen folgende Bedeutung haben:
R¹ ein Zuckersäurerest
R² Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können,
R³, R⁴ unabhängig voneinander Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder einen Zuckersäurerest,
für kosmetische Zubereitungen.

2. Verwendung von Zuckersäureureiden nach Anspruch 1, in der die Hydroxylfunktionen der Zuckersäuren in geschützter oder ungeschützter Form vorliegen.

3. Verwendung von Zuckersäureureiden nach den Ansprüchen 1 und 2 als Feuchthaltemittel.

4. Kosmetische Zubereitung, enthaltend eine wirksame Menge mindestens einer der Verbindungen der Formel I sowie übliche kosmetische Hilfs- und Zusatzstoffe.

5. Zuckersäureureide der Formel Ia, 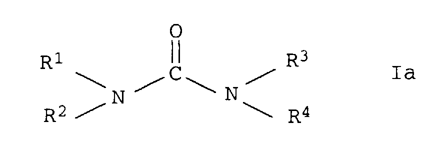 in der
R¹ für einen Zuckersäurerest aus der Gruppe Ribonsäure, Arabonsäure, Gluconsäure, Galactonsäure, Glucuronsäure, Galacturonsäure, Gulonsäure und Keto-gulonsäure steht,
R² Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl bedeutet, wobei die drei letztgenannten Reste substituiert sein können und
R³, R⁴ unabhängig voneinander für Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder für einen Zuckersäurerest aus der Gruppe Ribonsäure, Arabonsäure, Gluconsäure, Galactonsäure, Glucuronsäure, Galacturonsäure, Gulonsäure und Keto-gulonsäure stehen.

6. Zuckersäureureide nach Anspruch 5, in der die Hydroxylfunktionen der Zuckersäuren in geschützter oder ungeschützter Form vorliegen.

7. Verfahren zur Herstellung von Zuckersäureureiden der Formel I gemäß Anspruch 1, dadurch gekennzeichnet, daß man eine aktivierte Zuckersäure gegebenenfalls in Gegenwart einer tertiären Base mit Harnstoff oder einem Harnstoffderivat der Formel II, 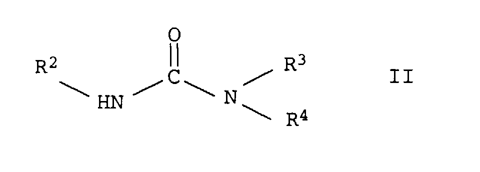 in der
R² Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl bedeutet, wobei die drei letztgenannten Reste substituiert sein können, und
R³, R⁴ unabhängig voneinander für Wasserstoff, C₁-C₂₀-Acyl-, C₁-C₂₀-Alkyl-, C₂-C₁₀-Alkenyl-, C₃-C₁₀-Cycloalkenyl-, C₃-C₁₀-Cycloalkylreste oder Aryl, wobei die drei letztgenannten Reste substituiert sein können, oder für einen Zuckersäurerest aus der Gruppe Ribonsäure, Arabonsäure, Gluconsäure, Galactonsäure, Glucuronsäure, Galacturonsäure, Gulonsäure und Keto-gulonsäure stehen, umsetzt.
